# EUROPEAN PATENT SPECIFICATION

(11) **EP 2 244 640 B1**
(45) Date of publication and mention of the grant of the patent: **30.03.2016**
(21) Application number: 08871500.8
(22) Date of filing: 16.12.2008
(51) Int. Cl.: A61B 17/122, A61B 17/128

(54) **RESOLUTION CLIP**
RESOLUTION-KLAMMER
PINCE DE RÉSOLUTION

(30) Priority: 22.01.2008 US 22586 P; 15.12.2008 US 334713 P
(43) Date of publication of application: 03.11.2010
(73) Proprietor: Boston Scientific Scimed, Inc., Maple Grove, MN 55311-1566 (US)
(72) Inventor: WEITZNER, Barry, Acton MA 01720 (US)
(74) Representative: Vossius & Partner Patentanwälte Rechtsanwälte mbB
(86) International application number: PCT/US2008/086981
(87) International publication number: WO 2009/094078

(56) References cited:
- WO-A-01/37742
- WO-A-96/00033
- WO-A-03/088825
- WO-A-2004/014435
- WO-A-2005/032381
- WO-A-2007/006140
- US-A1- 2004 193 189
- US-A1- 2007 038 233
- US-A1- 2007 149 988
- US-A1- 2008 004 637

## Description

### Priority Claim

This application claims the priority to the U.S. Provisional Application Serial No. 61/022,586, entitled "Resolution Clip" filed January 22, 2008(Pub. No.: US 2008/0306491 A1).

### Background

Advances in technology have led to a rise in the acceptance and practice of minimally invasive diagnostic procedures, such as advanced endoscopic and Natural Orifice Transluminal Endoscopic Surgical ("NOTES") procedures. Such procedures which may be performed with either no incisions or minimally sized incisions include, for example, surgical treatments within the brain, gastrointestinal tract, etc. as well as procedures for observing and/or collecting specimens from various organs, NOTES procedures may be employed without eternal incision as they enlist endoscope passed through natural orifices (i.e, the mouth, the anus, etc.) to access tissues within the body cavity via incisions through the lumenal walls, avoiding visible scars and the pain associated, for example, with the cutting of abdominal muscles.

Clips are one of the core tools for such minimally invasive surgeries, These clips arc used as closure devices to join the edges of natural or surgically created wounds to promote healing and stop bleeding, etc, The tissue joining forces applied by conventional clips are substantially concentrated at distal tips of the arms of the clips, spaced from the body of the clips allowing the clips, in certain cases, to slide along the tissue to which they are clamped.

US 2007/038233 A1, WO 96/00033 A, WO 03/088825, FR 2509601 and WO 2004/014435 disclose a surgical clip for temporary occluding a blood vessel, the clip having a pair of longitudinally extending members that are spring biased to a closed position.

WO 2007/006140 A discloses a bioactive clip for closing an aneurysm.

US 2004/193189 A1 discloses a clip having a generally U-shaped configuration with two parallel arms and a bridge connecting the arms. At least one resilient retainer extends rearward and from at least one of the arms and toward the other arm.

WO 01/37742 A discloses a clip similar to those known from US 2007/038233, the pair of longitudinally extending members, however, are spring biased to an open position.

US 2007/149988 A1 discloses a device for excluding the left atrial appendage (LAA) including first and second members, each with first and second ends. The second ends are connected by a hinge. The first ends interlock to close the device about the LAA.

US 2008/004637 A1 discloses a surgical clip including a pair of jaws, pivoted together, with a spring holding the jaws together in a closed position.

### Summary of the Invention

In some embodiments, the invention relates to systems for the sealing of a blood vessel, duct, etc. while the invention also relates to systems for sealing openings in vessels, ducts, etc. The invention provides a clip for compressing tissue comprising first and second legs, each extending from a distal end to a proximal end in combination with a joint coupled between proximal ends of the first and second legs and biasing the first and second legs into one of a closed configuration in which the first and second legs are separated from one another by a selected clipping distance and an open configuration in which the first and second legs are separated from one another by a tissue receiving distance greater than the clipping distance, the first and second legs being shaped so that, when in the closed configuration with no tissue received therebetween, a distance between the first and second legs is substantially constant from the joint to the distal ends of the first and second legs. In another embodiment, the invention related to systems for sealing and closing openings in the walls of vessels, ducts, etc.

### Brief Description of the Drawings

The accompanying drawing illustrates the design of the invention.
Fig. 1 shows a perspective view of a clip of an exemplary embodiment of the invention;
Fig. 2 shows a perspective view of a clip of a second exemplary embodiment of the invention;
Fig. 3 shows a perspective view of a lumen containing a fluid flow therethrough;
Fig. 4 shows a perspective view of a sealed lumen in accordance with an exemplary embodiment of the invention;

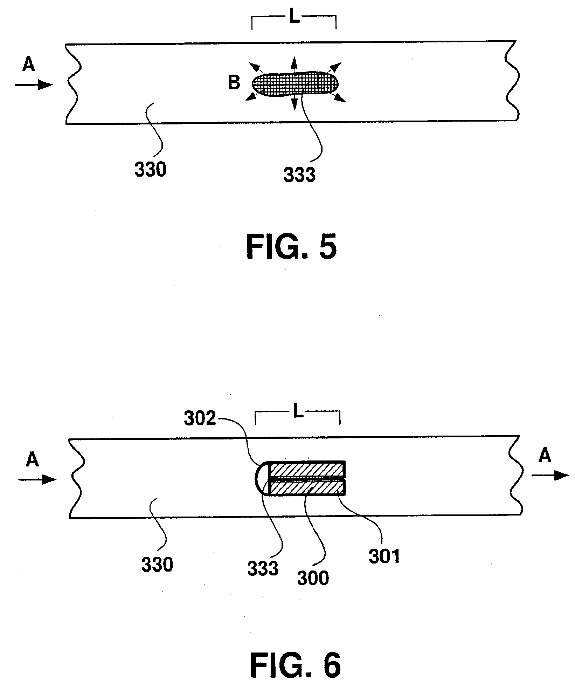

Fig. 5 shows a perspective view of a lumen containing a leakage source therein;
Fig, 6 shows a perspective view of a sealed lumen in accordance with an exemplary embodiment of the invention;
Fig. 7 shows a cross sectional view of a clip of a third exemplary embodiment of the present invention; and
Fig. 8 shows a close up view if a clip of the third exemplary embodiment in a closed configuration.

### Detailed Description

The invention which may be further understood with reference to the following description and the appended drawing relates to devices for clipping tissue during minimally invasive procedures.

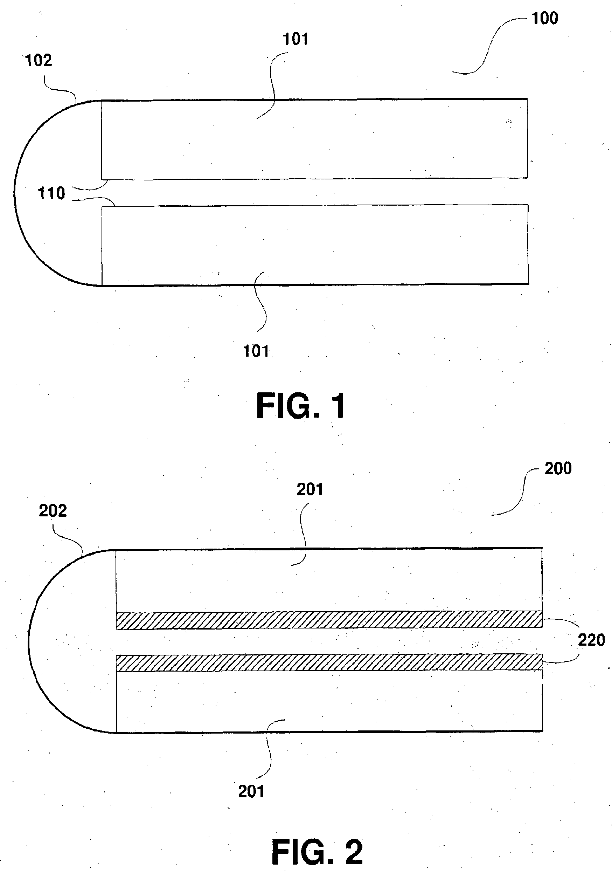

Devices according to the invention may employ clips which may include arms which, when closed, may approach one another along longitudinal sides thereof to apply a force to a length of tissue lying therebetween, As shown in Fig. 1 , a clip 100 according to an exemplary embodiment of the invention has a substantially U shaped configuration, wherein the legs 101 of the clip 100 are, when in a closed configuration, drawn toward one another substantially parallel to one another to apply a gripping force which remains substantially constant along the length of the legs 101. It is noted that the proximal legs of the clip 100 may alternatively be joined in a V configuration or may comprise a rectangularly shaped proximal connection. Furthermore, the binding portion 102 may comprise one of a smooth, unobstructed shape and an angular shape comprising a plurality of angled edges. In yet another alternate embodiment, the proximal ends of the legs 101 are joined directly to one another via a joint, wherein the joint means is known in the art. This particular embodiment may employ one of either two legs 101 joined proximally and a continuous construction, wherein the unibody structure comprises two leg portions extending outward from a proximal portion thereof.

Additionally, the proximal portion of the clip 100 may contain a locking arrangement which may be clamped together after insertion of the clip 100 onto target tissue, wherein the locking arrangement may be one known in the art such as, but not limited to, a latching mechanism, a friction fit lock, etc. Those skilled in the art will understand that the clip and locking arrangement are preferably designed to apply a desired compression force to the target tissue (e.g., sufficient to draw opposed portions of tissue together to prevent fluid flow therepast). A clip according to an exemplary embodiment of the invention may allow the legs 101 of the clip 100 to apply a constricting pressure to the constricted tissue substantially constant across the entire diameter of the tissue, blood vessel, duct or other luminal structure.

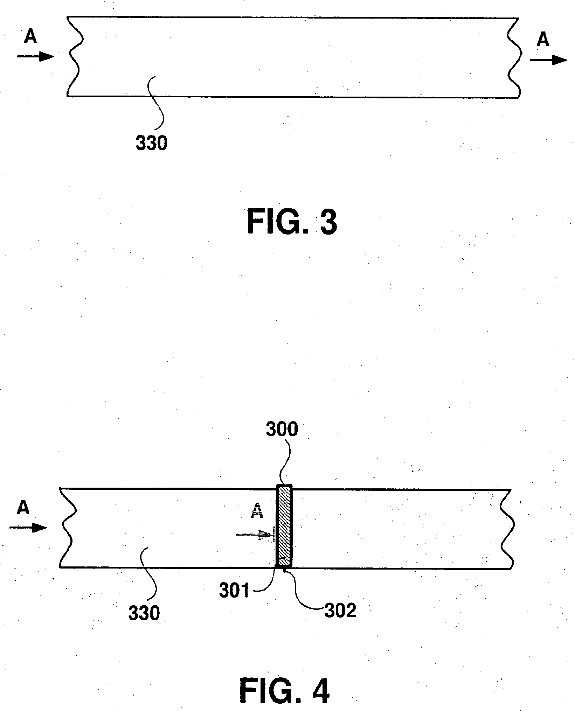

Although the clip 100 according to the invention is described in conjunction with an endoscope, those skilled in the art will understand that clips 100 according to the invention may be deployed with any insertion device (e.g., laparoscope, colonoscope, etc.). Accordingly, the design of the clip 100 may be altered as would be understood by those skilled in the art to render it suitable for selective engagement and disengagement from an endoscope or other insertion device. Furthermore, as would be understood by those skilled in the art, the dimensions of the clip 100 may be selected to enable deployment via the working channel of the endoscope or other minimally invasive means such as through a catheter sheath, along the outside of an endoscope via the employment of a guidewire, etc., as those skilled in the art will understand. In addition, a length of the legs of the clip 100 may be selected to extend along a length of tissue to be engaged. For example, where a hole in a vessel or duct is to be constricted and fluidly sealed, the length of the legs of the clip 100 may be at least equal to or greater than the longitudinal length of the hole, as will be described in greater detail below with respect to Figs. 3 and 4. Inner surfaces 110 of the legs 101 of the clip 100 according to this embodiment arc smooth and flat (i.e., substantially without protrusions) to minimize the formation of lesions on the clamped tissue. However, those skilled in the art will understand that, in cases where increased frictional engagement of clipped tissue is desired, one or both of the inner surfaces 110 of a clip 100 may be roughened or otherwise formed to increase the frictional engagement. As described below in regard to the clip 400, with the exception of the shape of the inner surfaces of the legs, the clips 100 and 400 and the apparatus for deploying these clips may be constructed substantially as described in U.S. 2008/0306491 A1, entitled Single Stage Hemostasis

### Clipping Device to Cohen et al.

The body clip 100 may be manufactured of a single piece of any suitable biocompatible material (i.e., a polymer, a metal, etc.), However, those skilled in the art will understand that other constructions may be equally suitable if not as economical to produce. It is further noted that a clip according to the present invention may be made substantially in accord with the construction of the clip described in U.S. 2008/0306491 A1 entitled "Single Stage Hemostatis Clipping Device," with the exception that the arms of the clip will be formed with inner surfaces thereof substantially smooth and parallel to one another so that, when in the closed configuration, these inner surfaces of the arms contact one another along an extended portion of the lengths thereof.

Fig. 2 shows a clip 200 according to a second exemplary' embodiment of the invention including a binding structure 202 attached to legs 201. Inner surfaces of the legs 101 are coated with a polymer 220 that is preferably substantially elastic to facilitate absorption of inward pressures in excess of the pressure desired to be applied to clipped tissue. Thus, the polymer 220 allows the clip 200 to obtain desired compressive properties while minimizing trauma to the clipped tissue. Accordingly, as the compressive force is distributed substantially evenly throughout the length of the clip 200, trauma such as cutting or scarring of tissue is minimized. Furthermore, by employing a substantially even distribution of pressure via the polymer 220, the clip 200 is restrained from slipping away from a desired tissue location. It is further noted that the thickness of the polymer 220 coat may vary from one of a minimal thickness required to coat a surface thereof to an increased thickness providing cushioned, tissue contacting surfaces,

Yet another embodiment comprises adding metal to one or both legs 101 of the clip 100 to fill gaps between the legs to enlarge an area over which force is applied by the clip 100 to the clipped tissue. As would be understood by those skilled in the art, increasing this area reduces trauma by spreading out the compressive force and enhances the sealing qualities of the clip 100 by eliminating uncompressed areas of tissue along the length of the legs 101, For example, when placed across a blood vessel, a clip 100 will apply force substantially equally across the diameter thereof eliminating areas through which flow may move past the clip 100. Similarly, when a clip 100 is applied to an opening in a blood vessel parallel to a longitudinal axis of the vessel to seal the opening, opposed edges of the opening will be held together along the entire length of the legs 101 and not just at distal ends thereof. In this particular embodiment, the clip may be manufactured in a manner similar to that described in the previously mentioned U.S. 2008/0306491 A1 with the distinct difference of providing an additional material along the length of the legs 101 to form opposed inner surfaces of the legs 101 shaped to contact one another along a longitudinal length thereof. As would be understood by those skilled in the art, the opposed inner surfaces of the legs 101 need not be flat and parallel to one another. Rather they need only have shapes which complement one another to mate in the closed configuration substantially without gaps therebetween.

In an alternate embodiment, a secondary filler material such as a plastic or silicone may be added to the legs 101 to provide the desired compression along the longitudinal length. It is noted that the design of the invention seeks to have as much of the longitudinal length of the legs 101 -contacting one another over as great an area as possible. However, as would be understood by those skilled in the art, design realities leave small gaps at the proximal and/or distal ends of the legs 101. However, the size and/or number of such gas is preferably minimized.

An exemplary embodiment of the clip of the present invention is shown in Figs, 3 and 4, Fig. 3 shows an unobstructed lumen 330 comprising a fluid (i.e., blood) flow A therethrough, wherein the flow A remains constant throughout the length of the lumen 330. In a preferred embodiment, a clip 300 according to the present invention may be employed to seal the lumen 330 to fluid flow, thereby preventing fluid from flowing therepast an attachment portion of the clip 300, as shown in Fig. 4. The clip 300 is placed thereover the tubular span of the lumen 330 in order tn seal the lumen 330. Accordingly, the flow A is halted at the attachment portion.

Specifically, it is noted that the legs 301 of the clip 330, due to their proximity to one another, cause the walls of the lumen 330 to constrict against one another, thereby preventing flow A therepast. The bearing surfaces of the legs 301 are formed so as to allow closure of the lumen 330 without crushing or damaging the lumen 330. As also noted earlier, a bearing surface of the clip 300 comprises the entire length of the clip and not just a distal tip thereof.

The clip 300 may also be used to seal an opening or incision in the lumen 330. Specifically, as shown in Figs, 5 and 6, a vessel 330 with a hole 333 formed in a wall thereof (e.g., during a medical procedure or as a result of trauma or tissue degradation) may be closed using a clip 300 according to a third embodiment of the invention to prevent fluid leakage and/or the introduction of foreign matter into the vessel 330. Accordingly, while fluid flow in the lumen 330 is directed along a longitudinal axis of the vessel 330 (e.g., in the direction of the arrow A), fluid may pass through the hole 333 and flow out as indicated by the arrows B with the many potential difficulties and dangers associated with such leaks. A clip 300 may be employed to seal the lumen 330 and prevent leakage therefrom. The clip 300 may be sized to be at least equal to the length L of the hole 333, so as to seal the entire length L thereof. Alternatively, more than one clip 300 may be used to seal a larger hole 333. The legs 301 of the clip 300 are moved to an open configuration and positioned engaging outlying walls on opposite sides of the hole 333. The legs 301 are then moved toward one another to the closed configuration to capture the perimeter of the hole 333 and draw the opposed edges of the tissue into sealing contact with one another closing off the leakage while permitting flow to continue through the vessel 330 in the direction of arrow A, As described earlier, a binding portion 302 of the clip 300 may draw the legs together to compress the captured tissue, as described earlier or, as in the case of the clips of U.S. 2008/0306491, the clip 300 may be biased toward an open position with a capsule slidable thereover to move the clip between the open and closed configurations and, eventually, to lock the clip 300 in the closed configuration. Accordingly, when placed thereupon a hole 333 on a lumen 330 or alternatively, on a vessel, duct, tissue or other luminal structure, the clip 300 serves to draw opposed portions of tissue together to fluidly seal an opening or lumen and/or to permit separated portions of tissue to heal to one another, etc.

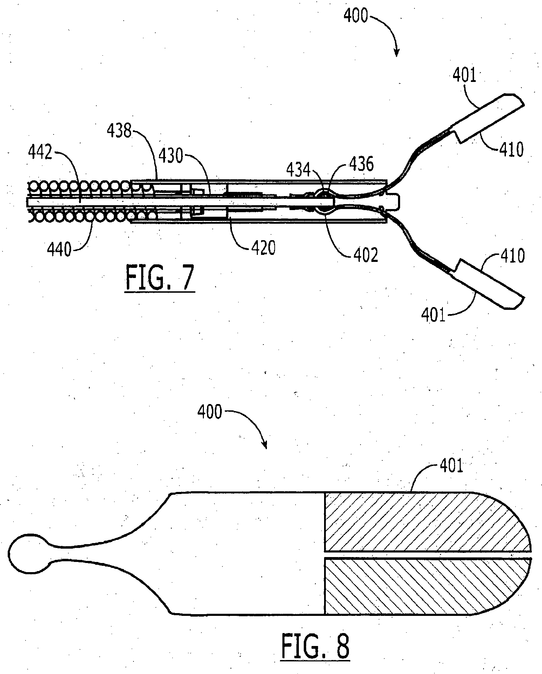

As shown in Fig. 7, a clip 400 may deploy from within a capsule 420. The clip 400 is a single piece hemostatic clip including a pair of tissue gripping legs 401 to clamp tissue. Each of the legs 401 contains thereupon a distal length thereof an increased thickness portion containing a bearing surface 410. The bearing surface 410 extends between 2.5 mm and 5.0 mm. along the length of each of the legs 401. Presently available clips comprise a bearing surface length of less than one mm. and are more often less than 0.2 mm. The exemplary embodiment of Fig. 7 provides an increased bearing surface length so as to minimize trauma to tissue gripped therebetween and to provide a gripping force for a larger area of tissue. During insertion, the legs 401 of the clip 400 are biased toward an open, tissue receiving configuration as shown in Fig, 7 and, when drawn proximally into the capsule 420, are constrained by the capsule 420 to remain in a closed configuration with the distal ends of the legs 401 brought together, as shown in Fig. 8. The capsule 420 is coupled to a bushing 430 which is further coupled to a handle (not shown) which remains outside the body via, for example, a flexible coil 440 which slidably receives a control wire 442 therethrough. The control wire 442, further connects the clip 400 to an actuator (not shown) on the handle. The coil 440 may be formed as a coil of wire or any other suitable hollow, flexible structure.

The capsule 420 further contains a deployment mechanism therein, the deployment mechanism including a cross bar 434 coupled to a distal end of the control wire 442, The control wire 442 passes through an opening in the proximal end of the clip 400 so that the cross bar 434 is received within a space 436 formed at the proximal end of the clip 400. Proximal movement of the control wire 442 therefore draws the clip 400 proximally. The cross bar 434 is coupled to the control wire 442 by a joint designed to fail when a desired load is applied to the control wire 442 through, for example, manipulation of the actuator. As the control wire 442 is drawn proximally, the legs 401 are drawn into the capsule 420 so that contact with the capsule 420 draws distal ends of the legs 401 toward one another, compressing any tissue located between the bearing surfaces 410. Furthermore, distal portions of the legs 402 are wider than proximal portions thereof, defining a maximum extent to which the legs 401 may be drawn into the capsule 420, Thus, as the control wire 442 is drawn proximally and the distal ends of the legs 401 approach one another, the force required to compress any tissue gripped thereby applies a load to the control wire 442 via the cross bar 434. After the legs 401 have been drawn into the capsule 420 to the maximum extent (i.e., when the portion of the legs contacting the distal end if the capsule 420 exceeds the diameter of the capsule 420, operating the actuator to draw the control wire 442 further proximally applies an increasing amount of force to the control wire 442 and, consequently, to the joint of the cross bar 434. When the predetermined load is reached and the joint 434 fails, the control wire 442 moves proximally relative to the cross bar 434 which remains trapped within the space 436 by inner walls of the capsule 420. A proximal end of the clip housed in the capsule 420 can then be locked in place via a series of tabs (not shown) located on the clip 400 which spring free when separated from the control wire 442 to engage corresponding openings in the capsule 420. This locks the clip 400 in position within the capsule 420 and maintains the legs 401 closed over any tissue gripped therebetween. A hypotube received around the distal end of the control wire 442 moves proximally with the control wire 442 to contact a bushing support 430 received in a proximal end of the capsule 420. When this bushing support 430 is driven proximally out of the capsule 420 into a bushing 438, tabs of the bushing 438, which had engaged openings in the proximal end of the capsule 420, spring inward out of engagement with the capsule 420 leaving the capsule 420 unattached and free to remain on the gripped tissue. The coil control wire 442 may then be partially or fully retracted into the coil 440 and the entire apparatus may be retracted from the body.

Alternate embodiments of the present invention may employ varying clip shapes, sizes and cross-sections, wherein each of these dimensions may be chosen based on a particular procedure to be performed. Alternatively, a plurality of coating materials and patterns may be employed on the legs of the clip. For example, instead of fully coating a surface of the legs, the coating material may be selectively placed only on portions thereof in any configuration. Furthermore, the clip may be modified to increase a surface area of the contacting surface of the legs, wherein the increased surface area may increase the retention capability of the clip thereupon a target tissue site. Furthermore, a greater surface area may allow for an increased ease of delivery of the clip to the target tissue site.

The invention has been described with reference to specific exemplary embodiments, Those skilled in the art will understand that various modifications and changes may be made to the embodiments. The specifications are, therefore, to be regarded in an illustrative rather than a restrictive sense,

## Claims

1. A clip (100) for compressing tissue, comprising:
first and second legs (101), each extending from a free distal end to a proximal end; and
a joint coupled between proximal ends of the first and second legs (101) and biasing the first and second legs (101) into one of a closed configuration in which the first and second legs (101) are separated from one another having a selected clipping distance and an open configuration in which the first and second legs (101) are separated from one another by a tissue receiving distance greater than the clipping distance,
**characterized in that**
the first and second legs (101) have a substantially parallel orientation to one another in the closed and open configurations,
the joint is configured to draw the first and second legs (101) toward one another to maintain the substantially parallel orientation,
a distance between inner surfaces of the first and second legs (101) when in the closed configuration is substantially constant and configured to apply a substantially constant gripping force along the length of the first and second legs (101); and
when in the closed configuration, the free distal end of each of the first and second legs is unconnected to one another.

2. The clip according to claim 1, wherein the clip (100) is formed of a first material with a coating of a second material over inner surfaces (110) of the first and second legs (101) which face one another.

3. The clip according to claim 2, wherein the second material has a greater compressibility than the first material or wherein the second material is no more compressive than the first material.

4. The clip according to claim 3, wherein the first material is a metal and the second material is a polymer (220).

5. The clip according to claim 1, wherein shapes of the inner surfaces (110) of the first and second legs (101) complement each other.

6. The clip according to claim 3, wherein the inner surfaces (110) of the first and second legs (101) are substantially flat and parallel to one another.

7. The clip according to claim 2, wherein the joint includes a binding portion (102) formed of the first material coupled between proximal ends of the first and second legs (101).

8. The clip according to claim 6, wherein the binding portion (102) is biased to draw the first and second legs (101) to the closed configuration, or wherein the binding portion (102) is biased to draw the first and second legs (101) toward the open configuration.

9. The clip according to claim 6, wherein a width of the binding portion (102) is less than a width of the inner surfaces (110) of the first and second legs (101).

10. The clip according to claim 2, wherein the coating is made of one of a compressible polymer (220), a compressible plastic and a compressible silicone.

11. The clip according to claim 2, wherein the coating completely or partially covers the inner surface (110) of the first leg.

12. The clip according to claim 1, wherein the first and second legs (101) and the joint are integrally formed.

13. The clip according to claim 1, wherein the inner surface (110) of the first leg is roughened to increase frictional engagement with tissue.

## Patentansprüche

1. Klammer (100) zum Komprimieren von Gewebe, die aufweist:
einen ersten und einen zweiten Schenkel (101), die sich jeweils von einem freien distalen Ende zu einem proximalen Ende erstrecken; und
ein Gelenk, das zwischen proximalen Enden des ersten und zweiten Schenkels (101) gekoppelt ist und den ersten und zweiten Schenkel (101) in eine geschlossene Konfiguration, in der der erste und zweite Schenkel mit einem ausgewählten Klemmabstand voneinander getrennt sind, oder eine offene Konfiguration vorspannt, in der der erste und zweite Schenkel (101) mit einem Gewebe aufnehmenden Abstand voneinander getrennt sind, der größer als der Klemmabstand ist,
**dadurch gekennzeichnet, dass**
der erste und zweite Schenkel (101) in der geschlossenen und offenen Konfiguration eine im Wesentlichen parallele Orientierung zueinander haben,
das Gelenk so konfiguriert ist, dass es den ersten und zweiten Schenkel (101) zueinander zieht, um die im Wesentlichen parallele Orientierung beizubehalten,
ein Abstand zwischen Innenflächen des ersten und zweiten Schenkels (101) in der geschlossenen Konfiguration im Wesentlichen konstant und so konfiguriert ist, dass eine im Wesentlichen konstante Greifkraft über die Länge des ersten und zweiten Schenkels (101) ausgeübt wird; und
in der geschlossenen Konfiguration das freie distale Ende jeweils des ersten und zweiten Schenkels nicht miteinander verbunden ist.

2. Klammer nach Anspruch 1, wobei die Klammer (100) aus einem ersten Material mit einer Beschichtung aus einem zweiten Material über Innenflächen (110) des ersten und zweiten Schenkels (101) gebildet ist, die zueinander weisen.

3. Klammer nach Anspruch 2, wobei das zweite Material eine größere Komprimierbarkeit als das erste Material hat oder wobei das zweite Material nicht stärker als das erste Material zu komprimieren ist.

4. Klammer nach Anspruch 3, wobei das erste Material ein Metall ist und das zweite Material ein Polymer (220) ist.

5. Klammer nach Anspruch 1, wobei Formen der Innenflächen (110) des ersten und zweiten Schenkels (101) einander ergänzen.

6. Klammer nach Anspruch 3, wobei die Innenflächen (110) des ersten und zweiten Schenkels (101) im Wesentlichen flach und parallel zueinander sind.

7. Klammer nach Anspruch 2, wobei das Gelenk einen aus dem ersten Material gebildeten Verbindungsabschnitt (102) aufweist, der zwischen proximalen Enden des ersten und zweiten Schenkels (101) gekoppelt ist.

8. Klammer nach Anspruch 6, wobei der Verbindungsabschnitt (102) so vorgespannt ist, dass er den ersten und zweiten Schenkel (101) in die geschlossene Konfiguration zieht, oder wobei der Verbindungsabschnitt (102) so vorgespannt ist, dass er den ersten und zweiten Schenkel (101) in die offene Konfiguration zieht.

9. Klammer nach Anspruch 6, wobei eine Breite des Verbindungsabschnitts (102) kleiner als eine Breite der Innenflächen (110) des ersten und zweiten Schenkels (101) ist.

10. Klammer nach Anspruch 2, wobei die Beschichtung aus einem komprimierbaren Polymer (220), einem komprimierbaren Kunststoff oder einem komprimierbaren Silikon hergestellt ist.

11. Klammer nach Anspruch 2, wobei die Beschichtung die Innenfläche (110) des ersten Schenkels vollständig oder teilweise bedeckt.

12. Klammer nach Anspruch 1, wobei der erste und zweite Schenkel (101) und das Gelenk integral ausgebildet sind.

13. Klammer nach Anspruch 1, wobei die Innenfläche (110) des ersten Schenkels aufgeraut ist, um Reibeingriff mit Gewebe zu verstärken.

## Revendications

1. Pince (100) pour comprimer du tissu, comprenant :
des première et seconde pattes (101), chacune s'étendant à partir d'une extrémité distale libre à une extrémité proximale ; et
une articulation couplée entre les extrémités proximales des première et seconde pattes (101) et sollicitant les première et seconde pattes (101) dans l'une parmi une configuration fermée dans laquelle les première et seconde pattes (101) sont séparées l'une de l'autre ayant une distance de pincement sélectionnée et une configuration ouverte dans laquelle les première et seconde pattes (101) sont séparées l'une de l'autre par une distance de réception de tissu supérieure à la distance de pincement,
**caractérisée en ce que** :
les première et seconde pattes (101) ont une orientation sensiblement parallèle entre elles dans les configurations fermée et ouverte,
l'articulation est configurée pour tirer les première et seconde pattes (101) l'une vers l'autre afin de maintenir l'orientation sensiblement parallèle,
une distance entre les surfaces internes des première et seconde pattes (101) lorsqu'elle est dans la configuration fermée, est sensiblement constante et configurée pour appliquer une force de préhension sensiblement constante le long de la longueur des première et seconde pattes (101) ; et
lorsqu'elle est dans la configuration fermée, l'extrémité distale libre de chacune des première et seconde pattes n'est pas raccordée à l'autre.

2. Pince selon la revendication 1, dans laquelle la pince (100) est formée à partir d'un premier matériau avec un revêtement d'un second matériau sur les surfaces internes (110) des première et seconde pattes (101) qui se font face.

3. Pince selon la revendication 2, dans laquelle le second matériau a une compressibilité plus importante que le premier matériau ou dans laquelle le second matériau n'est pas plus compressible que le premier matériau.

4. Pince selon la revendication 3, dans laquelle le premier matériau est un métal et le second matériau est un polymère (220).

5. Pince selon la revendication 1, dans laquelle les formes des surfaces internes (110) des première et seconde pattes (101) se complètent.

6. Pince selon la revendication 3, dans laquelle les surfaces internes (110) des première et seconde pattes (101) sont sensiblement plates et parallèles entre elles.

7. Pince selon la revendication 2, dans laquelle l'articulation comprend une partie de liaison (102) formée avec le premier matériau, couplée entre les extrémités proximales des première et seconde pattes (101).

8. Pince selon la revendication 6, dans laquelle la partie de liaison (102) est sollicitée pour tirer les première et seconde pattes (101) dans la configuration fermée ou bien dans laquelle la partie de liaison (102) est sollicitée pour tirer les première et seconde pattes (101) vers la configuration ouverte.

9. Pince selon la revendication 6, dans laquelle une largeur de la partie de liaison (102) est inférieure à une largeur des surfaces internes (110) des première et seconde pattes (101).

10. Pince selon la revendication 2, dans laquelle le revêtement est réalisé avec l'un parmi un polymère compressible (220), un plastique compressible et une silicone compressible.

11. Pince selon la revendication 2, dans laquelle le revêtement couvre complètement ou partiellement la surface interne (110) de la première patte.

12. Pince selon la revendication 1, dans laquelle les première et seconde pattes (101) et l'articulation sont formées de manière solidaire.

13. Pince selon la revendication 1, dans laquelle la surface interne (110) de la première patte est rendue rugueuse pour augmenter la mise en prise par friction avec le tissu.
